# EUROPEAN PATENT SPECIFICATION

(11) **EP 2 481 443 B1**
(45) Date of publication and mention of the grant of the patent: **25.06.2014**
(21) Application number: 12152358.3
(22) Date of filing: 25.01.2012
(51) Int. Cl.: A61M 35/00, A61K 9/70, A61F 13/02

(54) **Patch**
Flicken
Timbre

(30) Priority: 26.01.2011 JP 2011014425
(43) Date of publication of application: 01.08.2012
(73) Proprietor: Nitto Denko Corporation, Ibaraki-shi, Osaka 567-8680 (JP)
(72) Inventor: Aoyagi, Kazuhiro, Ibaraki-shi, Osaka 567-8680 (JP); Iwao, Yoshihiro, Ibaraki-shi, Osaka 567-8680 (JP); Matsuoka, Kensuke, Ibaraki-shi, Osaka 567-8680 (JP); Tanaka, Tomoya, Ibaraki-shi, Osaka 567-8680 (JP)
(74) Representative: Grünecker, Kinkeldey, Stockmair & Schwanhäusser

(56) References cited:
- EP-A1- 1 915 976
- JP-U- 50 123 187
- US-A- 5 052 381

## Description

### FIELD OF THE INVENTION

The present invention relates to a sheet-shaped patch including a pressure-sensitive adhesive layer on one surface of a backing to provide a pressure-sensitive adhesive surface showing adhesiveness at ordinary temperature and further including a release liner covering the pressure-sensitive adhesive surface. More specifically, the present invention relates to a sheet-shaped patch where the release liner is formed in a state of being splittable by a parting line.

### BACKGROUND OF THE INVENTION

Conventionally, a sheet-shaped patch to be attached to skin for protecting the skin surface or percutaneously administering a drug has been developed.
In such a patch, a pressure-sensitive adhesive layer is usually formed on one surface of a backing using a pressure-sensitive adhesive showing adhesiveness at ordinary temperature, and a release liner covering the pressure-sensitive adhesive surface provided by the pressure-sensitive adhesive layer is further provided so as to protect the pressure-sensitive adhesive surface with the release liner.
Regarding this kind of patch, if the release liner is entirely removed before attaching the patch to the desired portion, a finger adheres to the pressure-sensitive adhesive surface and the handling usually becomes difficult. Therefore, this kind of patch is usually designed so that the release liner can be split and partially removed and after attaching the exposed pressure-sensitive adhesive surface portion to the desired portion while taking hold the remaining portion of the release liner, the entire pressure-sensitive adhesive surface can be attached to the desired portion by removing the remaining release liner.

For example, International Publication No. WO00/69422 describes a technique of forming, in the release liner, a cut line having a predetermined shape and extending from one edge part of the release liner to the other opposing edge part so that a part of the release liner can be separated ahead of the remaining part of the release liner.

However, when the patch is produced in the form of the release liner being completely split before use as in International Publication No. WO00/69422, the cut line may expand upon application of slight stress and the pressure-sensitive adhesive surface may be exposed through a gap in the cut line.

This brings about a fear that the patch may attach to an unintended portion before use or dusts or the like may adhere to the protruded pressure-sensitive adhesive to provide an unhygienic state.

Also, in a case where a drug to be percutaneously administered is incorporated in the pressure-sensitive adhesive layer, exposure of the pressure-sensitive adhesive surface may cause the drug to bleed out of the exposed surface.

European Patent Application No. 1 915 976 A discloses a patch essentially comprising a stretchable support and release liner having a division zone which is constructed in a pattern that allows the release liner to be divided by pulling the entire patch apart. Whereas such a construction enables to easily detach the release sheet, it is not suitable for patches with non-stretchable supports, wherein force alongside the splitting direction has to be applied in order to divide the release liner.

Furthermore, JP-A-2008-19209 (the term "JP-A" as used herein means an "unexamined published Japanese patent application") discloses a patch where a groove with a predetermined depth is provided in a release liner, and it is described that a parting line for guiding the cutting position and putting the release liner into a desired split state is formed by the groove.

That is, in the patch of JP-A-2008-19209, the release liner is cut along the bottom of the groove and a cut line is not formed in the release liner before use, so that the pressure-sensitive adhesive surface can be usually kept from being exposed before use.
However, in order to attain easy splitting of the release liner, control of the thickness of the release liner in the bottom part plays an important factor and depending on the depth of the groove formed, it may become difficult to cut the release liner along the groove.

That is, conventional patches have a problem that it is difficult to keep the pressure-sensitive adhesive surface from being largely exposed due to slight stress before use and at the same time, easily split the release liner when using the patch.

### SUMMARY OF THE INVENTION

In view of these problems, an object of the present invention is to provide a patch ensuring that the pressure-sensitive adhesive surface can be kept from being largely exposed due to slight stress before use and at the same time, the release liner can be easily split when using the patch.

In order to attain the above-described object, the present invention provides a sheet-shaped patch comprising: a backing; a pressure-sensitive adhesive layer provided on one surface of the backing, the pressure-sensitive adhesive layer providing a pressure-sensitive adhesive surface showing an adhesiveness at ordinary temperature; and a release liner covering the pressure-sensitive adhesive surface, wherein the release liner has a parting line constituted of cut lines and connection parts provided alternately, and is formed to be splittable into a first region and a second region by sequentially cutting the connection parts along the parting line, thereby enabling separation of the first region from the pressure-sensitive adhesive surface ahead of the second region while performing cutting of the connection parts, and wherein the cut lines each have an upstream end part and a downstream end part in a splitting direction, and the cut lines are disposed such that the downstream end parts are aligned along the splitting direction and each upstream end part is arranged to be located on a first region side relative to each corresponding downstream end part, so that, at the time of splitting the first region from the second region, one of the connection parts which is located between one of the cut lines located on an upstream side in the splitting direction and another one of the cut lines adjacent to a downstream side of the one of the cut lines can be cut in a form where the downstream end part of the one of the cut lines and a midway portion between the upstream end part and the downstream end part of the another one of the cut lines are connected and a cutting direction connecting the downstream end part of the one of the cut lines and the midway portion of the another one of the cut lines can be inclined toward the first region with respect to the splitting direction.

According to the present invention, a parting line is formed in the release liner by alternately providing cut lines and connection parts, so that the cut lines can be kept from being largely opened due to slight stress.
Incidentally, only such a function may be attained by forming a dashed parting line called "perforation" or the like composed of short linear cut lines. However, when splitting the release liner, a user usually performs an operation of sequentially cutting the connection parts from one end of the parting line and therefore, if a dashed parting line is merely formed, splitting along the parting line may require a careful operation.
That is, the force applied to the connection part when splitting the release liner and first separating a part of the release liner from the pressure-sensitive adhesive surface takes a form of creating an angle slightly shifted to right side when the user is right-handed, and shifted to left side when the user is left-handed, with respect to the parting line direction and thus, the cutting direction of connection parts is liable to be shifted with respect to the parting line direction. Therefore, when a dashed parting line is merely formed, splitting of the release liner along the parting line cannot be sufficiently easy.

On the other hand, in the present invention, the cut lines are disposed by aligning the downstream end parts along the splitting direction (dividing direction) and arranging each upstream end part to be located on the first region side relative to each corresponding downstream end part, so that a connection part between one of the cut lines (first cut line) on the upstream side in the splitting direction and another one of the cut lines (second cut line) adjacent to the downstream side of the one of the cut lines (first cut line) can be cut in a form where the downstream end part of the one of the cut lines (first cut line) and a midway portion between the upstream end part and the downstream end part of the another one of the cut lines (second cut line) are connected and the cutting direction connecting the downstream end part of the one of the cut lines (first cut line) and the midway portion of the another one of the cut lines (second cut line) can be inclined toward the region firstly separated with respect to the splitting direction. Therefore, the release liner can be easily split along the parting line (hereinafter also may referred as splitting line or dividing line).
That is, even when the cutting direction of connection parts is shifted due to the manner of applying the natural force by the dominant hand, since the next cut line is positioned in that direction, adjacent cut lines are readily connected.

As described above, according to the patch of the present invention, not only the pressure-sensitive adhesive surface can be kept from being largely exposed due to slight stress before use but also the release liner can be easily split when using the patch.

In one embodiment of the patch of the present invention, each of the cut lines includes a first segment locating a starting point at the upstream end part and a terminal point at the downstream end part or at a point traced back from the downstream end part in a direction opposite the splitting direction, and the cut lines are disposed such that the first segments of the cut lines adjacent to each other are provided substantially in parallel.
Herein, each of the cut lines may include the first segment locating the starting point at the upstream end part and the terminal point at the point traced back from the downstream end part in the direction opposite the splitting direction and further include a second segment extending from the terminal point of the first segment to the downstream end part.
In addition, each of the cut lines may further include, in addition to the first and second segments, a third segment locating a starting point at the terminal point of the first segment and a terminal point on an upstream side relative to the starting point thereof and also on the second region side opposite the first segment, where the first segment, the second segment and the third segment are disposed in a Y-shaped manner.

In another embodiment of the patch of the present invention, each of the cut lines has a maximum width of from 1 to 120 µm.

In still another embodiment of the patch of the present invention, each of the cut lines has a total length of from 0.1 to 60 mm.

In still another embodiment of the patch of the present invention, the parting line enables splitting the release liner into substantially equal areas.

### BRIEF DESCRIPTION OF THE DRAWINGS

Fig. 1 is a perspective view showing a patch in one embodiment.
Fig. 2A is a plan view of the patch of Fig. 1, Fig. 2B is an enlarged plan view enlarging the circular region surrounded by a virtual line X in Fig. 2A, and Fig. 2C is a cross-sectional view cut along Y-Y line in Fig. 2B.
Figs. 3A to 3D are plan views showing patches in other embodiments.
Fig. 4A is a plan view showing a patch outside the scope of the present invention, and Fig. 4B is an enlarged plan view enlarging the circular region surrounded by a virtual line X' in Fig. 4A.
Figs. 5A and 5B are plan views showing patch outside the scope of the present invention.
Figs. 6A to 6C are plan views showing the outline of the evaluation method in Examples.

### Description of Reference Numerals and Signs

- 1: Patch
- 10: Backing
- 20: Pressure-sensitive adhesive layer
- 20a: Pressure-sensitive adhesive surface
- 30: Release liner
- 30a: First region
- 30b: Second region
- 31: Cut line
- 32: Connection part
- 311: First segment
- 312: Second segment
- 313: Third segment
- C: Parting line
- D1: Splitting direction

### DETAILED DESCRIPTION OF THE INVENTION

Preferred embodiments for carrying out the present invention are described below by referring to the drawings. Furthermore, in the drawing referred to in the following detailed description, for facilitating the understanding, the cut line is shown by employing a length, a width and the like different from those in the actual patch and in other configurations, the dimension in the actual patch is also not always strictly denoted.

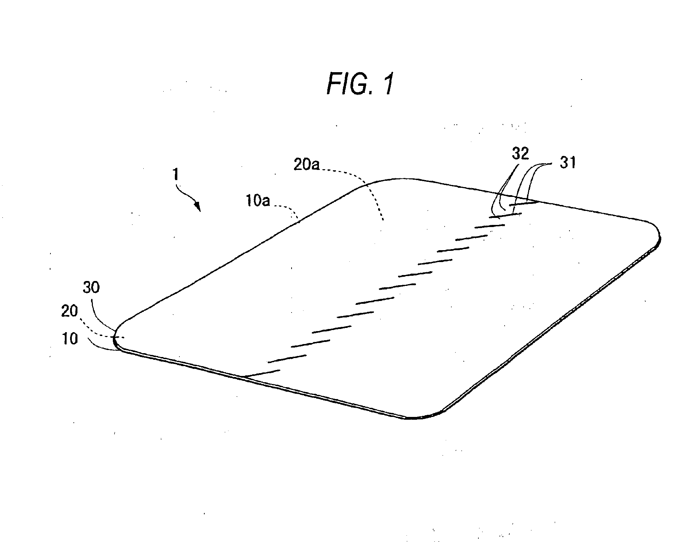

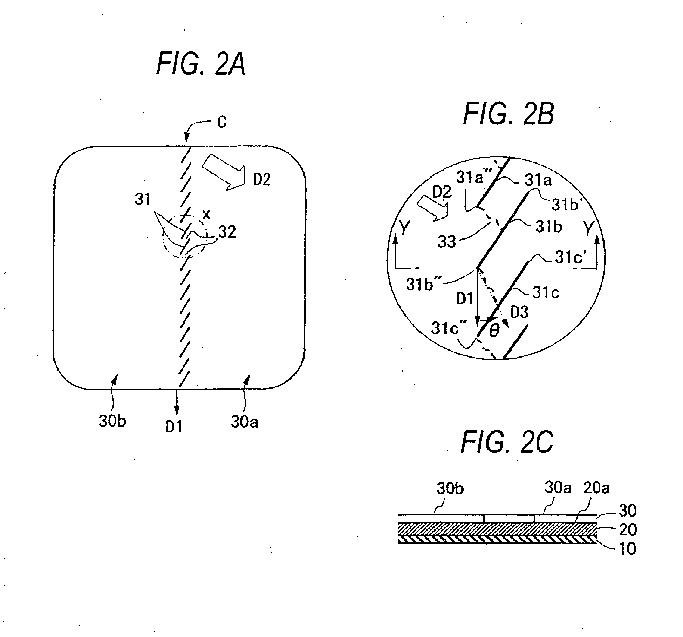

The patch according to this embodiment is described by referring to Figs. 1 to 2C. As shown in these Figures, the sheet-shaped patch 1 (sheet-like patch) according to this embodiment includes, on one surface of a backing 10, a pressure-sensitive adhesive layer 20 which provides a pressure-sensitive adhesive surface 20a showing adhesiveness at ordinary temperature (for example, 25°C) and further includes a release liner 30 which covers the pressure-sensitive adhesive surface 20a.
In the release liner 30, a parting line C is formed by alternately providing cut lines 31 and connection parts 32 so that the release liner can be split into a first region 30a and a second region 30b by sequentially cutting the connection parts 32 along the parting line C in the splitting direction (arrow D1 in Fig. 2), and the first region 30a is separated while performing the splitting of the release liner, whereby the split first region 30a can be separated from the pressure-sensitive adhesive surface 20a ahead of the second region 30b.

Describing more specifically, in the patch 1 of the present invention illustrated in Figs. 1 to 2C, a sheet having a nearly square planar view is employed for the backing 10, and a square sheet with four corners being arced (R is provided) is employed.
Incidentally, in this embodiment, the patch 1 is described by referring to, for example, a case where the shape in a planar view is square as above, but the shape may be other quadrilateral shapes such as rectangle and trapezoid, may be other polygonal shapes such as triangle and pentagon, or may be a circular, elliptical, amorphous or the like shape.
In a case where the shape is a polygonal shape, the apex may be of angular shape or imparted with slight R as illustrated in the Figures.
The size of the patch is not particularly limited, but the area is, for example, approximately from 25 to 40,000 mm², preferably from 100 to 10,000 mm².
In a case where the planar shape of the patch is rectangular, the length of one side is, for example, from 5 to 200 mm, preferably from 10 to 100 mm.

The pressure-sensitive adhesive layer 20 may be formed so as to cover the entire surface of the backing 10 or cover only a part thereof.
For example, it is also possible to more facilitate the operation of removing the release liner 30 by forming the release liner 30 nearly in the same shape as the backing 10 and forming the pressure-sensitive adhesive layer 20 to have a shape giving a square planar view slightly smaller than the backing 10 and the release liner 30 and thereby provide, in the outer circumferential part of the patch 1, a region where the release liner 30 is not adhered to the pressure-sensitive adhesive surface 20a.
Alternatively, the release liner 30 may be formed to have a shape slightly larger than those of the backing 10 and the pressure-sensitive adhesive layer 20 (hereinafter, both of the backing and the pressure-sensitive adhesive layer are sometimes collectively referred to as a "patch main body") and thereby let the outer circumferential part of the release liner project beyond the patch main body.
In a case of employing such an embodiment, even when the pressure-sensitive adhesive layer is provided on the entire surface of the backing and the outer edge part of the pressure-sensitive adhesive layer is exposed in the outer circumference of the patch main body, the projected portion of the release liner can prevent a member or the like of some kind from contacting with the exposed portion.
For example, in the case of housing the patch in a packaging, a distance corresponding to the projection length of the release liner is ensured between the exposed portion of the pressure-sensitive adhesive layer and the inner surface of the packaging and even when a component of the pressure-sensitive adhesive bleeds out from the pressure-sensitive adhesive layer, the bled component can be restrained from attaching to the inner surface of the packaging.
Accordingly, the patch can be prevented from sticking to the inner surface of the packaging due to the bled component, and the patch can be thus easily taken out from the package.
Also, thanks to projection of the release liner, when separating the release liner from the pressure-sensitive adhesive surface of the patch main body, the release liner can be easily caught and in turn, the separation operation can be facilitated.
Incidentally, the release liner is preferably adjusted, at the time of determining its shape, to intersect the outer edge with a cut line and create a state where an incision acting as a starting point of splitting is provided by the cut line. For example, in a case where a resin film having formed therein a cut line is punched out to form a release liner, the resin film is preferably punched out to cut between one end part and another end part of a cut line.

In the patch 1 according to this embodiment, the backing 10 is not particularly limited, and various kinds of plastic films, nonwoven fabrics, paper, woven fabrics, knitted fabrics, metal foils, and a laminate thereof may be employed.
If desired, the material mentioned above may be subjected to metal deposition and then employed as the backing 10.

The plastic film is not particularly limited, and examples thereof include various films composed of: a polyvinyl chloride alone; a copolymer material of a monomer such as ethylene, propylene, vinyl acetate, acrylic acid, acrylic acid ester, methacrylic acid, methacrylic acid ester, acrylonitrile, styrene and vinylidene chloride, and other monomers; an olefin-based polymer such as polyethylene, polypropylene and ethylene-vinyl acetate copolymer; a polyester-based polymer such as polyethylene terephthalate and polyether polyester; or a polyamide-based polymer such as polyether polyamide block polymer.

The thickness of the backing 10 may be, for example, from 5 to 500 µm and is preferably from 10 to 200 µm.

The pressure-sensitive adhesive layer 20 is formed in a layer form from a pressure-sensitive adhesive having adhesiveness at ordinary temperature (25°C).
The pressure-sensitive adhesive is not particularly limited, and examples thereof include an acrylic pressure-sensitive adhesive composed of an acryl-based polymer; a rubbery pressure-sensitive adhesive including a styrene-diene-styrene block copolymer (e.g., styrene-isoprene-styrene block copolymer, styrene-butadiene-styrene block copolymer), a polyisoprene, a polyisobutylene or a polybutadiene; a silicone-based pressure-sensitive adhesive including a silicone rubber, a dimethylsiloxane base or a diphenylsiloxane base; a vinyl ether-based pressure-sensitive adhesive such as polyvinyl methyl ether, polyvinyl ethyl ether and polyvinyl isobutyl ether; a vinyl ester-based pressure-sensitive adhesive such as vinyl acetate-ethylene copolymer; and a polyester-based pressure-sensitive adhesive composed of a carboxylic acid component such as dimethyl terephthalate, dimethyl isophthalate and dimethyl phthalate, and a polyhydric alcohol component such as ethylene glycol.

The pressure-sensitive adhesive layer may also be formed from a pressure-sensitive adhesive which further contains any kinds of various organic liquid components to the pressure-sensitive adhesive mentioned above.
From the standpoint that a soft texture on attaching the patch to skin can be imparted to the pressure-sensitive adhesive layer, an acrylic pressure-sensitive adhesive or a rubbery pressure-sensitive adhesive is preferred, and from the standpoint that crosslinking is facilitated and a large amount of liquid component can be held in the pressure-sensitive adhesive layer, an acrylic pressure-sensitive adhesive is preferred.

Particularly preferred examples of the rubbery pressure-sensitive adhesive include a rubbery pressure-sensitive adhesive whose main component is at least one member selected from polyisobutylene, polyisoprene and a styrene-diene-styrene copolymer.
Also, the particularly preferred examples of the acrylic pressure-sensitive adhesive include an acrylic acid ester-based pressure-sensitive adhesive whose main component is a polymer containing a C₂₋₁₈ alkyl (meth)acrylate as a polymerization component.

The organic liquid component to be incorporated into the pressure-sensitive adhesive layer is not particularly limited, and an organic liquid component having an effect of accelerating percutaneous absorption is preferred.
Examples of such an organic liquid component include glycols such as ethylene glycol, diethylene glycol, propylene glycol, triethylene glycol, polyethylene glycol and polypropylene glycol; fats and oils such as olive oil, castor oil, squalane and lanoline; hydrocarbons such as liquid paraffin; various surfactants; an ethoxylated stearyl alcohol; glycerin monoesters such as oleic acid monoglyceride, caprylic acid monoglyceride and lauryl acid monoglyceride, glycerin diesters, glycerin triesters, and a mixture thereof; fatty acid alkyl esters such as ethyl laurate, isopropyl myristate, isotridecyl myristate, octyl palmitate, isopropyl palmitate, ethyl oleate and diisopropyl adipate; higher fatty acids such as oleic acid and caprylic acid; N-methylpyrrolidone; and 1,3-butanediol.

In a case where an organic liquid component is contained in the pressure-sensitive adhesive layer, the organic liquid component or the like may protrude from the cut line formed in the release liner or the marginal part of the patch before use. In this connection, the patch 1 according this embodiment where the connection part 32 can keep the cut line 31 from being enlarged due to slight stress is particularly effective.

From such a standpoint, the content of the organic liquid component in the pressure-sensitive adhesive layer 20 is preferably from 5 to 70 mass%, more preferably from 10 to 65 mass%, and most preferably from 15 to 60 mass%, based on the total mass of the pressure-sensitive adhesive layer 20.
Also, from the same standpoint, the thickness of the pressure-sensitive adhesive layer 20 is preferably from 20 to 300 µm, more preferably from 30 to 250 µm, and most preferably from 50 to 200 µm.

In addition, a drug may be further incorporated into the pressure-sensitive adhesive layer 20.
A patch having a pressure-sensitive adhesive layer containing a drug is sometimes referred to as a "patch preparation" in particular, and the term "patch" as used in the present specification is used with intent to include such a patch preparation.
The drug incorporated into the pressure-sensitive adhesive layer 20 is not particularly limited, and a percutaneously absorbable drug which can be administered to a mammal such as human through its skin is preferred.

Specific examples of the drug include general anesthetic, hypnotic sedative, antiepileptic drug, antipyretic anti-inflammatory analgesic, seasick remedy, psychoneurotic agent, central nervous agent, antidementia agent, local anesthetic, skeletal muscle relaxant, autonomic nervous system drug, spasmolytic agent, antiparkinsonian drug, antihistamine agent, cardiac stimulant, anti-arrhythmic, diuretic, hypotensive agent, vasoconstrictor, coronary vasodilator, peripheral vasodilator, anti-arteriosclerotic agent, cardiovascular preparation, respiratory stimulant, antitussive/expectorant, hormonal drug, external medicine for suppurative disease, analgesic-antipruritic-astringent-anti-inflammatory, drug for parasitic skin disease, haemostatic, gout remedy, diabetes drug, antineoplastic agent, antibiotic, chemotherapeutic, narcotic, and stop smoking aid.

The drug can be contained in the pressure-sensitive adhesive layer in an amount referred to as an effective dose in the present specification, which is an amount high enough to bring about a desired result, for example, a desired therapeutic outcome, in the treatment of disease, condition or disorder.
The effective dose of drug means, for example, a drug which is nontoxic but in an amount sufficiently large to bring a selected effect over a specific time, and such an amount can be appropriately determined.

The amount of the drug in the pressure-sensitive adhesive layer is not particularly limited as long as it is in the range satisfying the effect of the drug for percutaneous absorption and not impairing the adhesion property of the pressure-sensitive adhesive, but the amount may be, for example, from 0.1 to 60 mass%, preferably from 0.5 to 40 mass%, based on the total mass of the pressure-sensitive adhesive layer.
If the amount of the drug is less than 0.1 mass%, the therapeutic outcome may be insufficient, whereas if it exceeds 60 mass%, the content of the pressure-sensitive adhesive component constituting the pressure-sensitive adhesive layer is reduced and this may lead to a failure in obtaining sufficient skin adhesion or may give rise to an economic disadvantage.

The release liner 30 for protecting the pressure-sensitive adhesive surface 20a of the pressure-sensitive adhesive layer 20 until using the patch 1 is not particularly limited, but examples thereof include a plastic film such as polyethylene terephthalate (PET) film, polyester film other than PET film, polyimide film, polypropylene film, polyethylene film and polycarbonate film; a metal-deposited plastic film obtained by vapor-depositing a metal on the plastic film; papers such as Japanese paper, paper, craft paper, glassine paper and wood-free paper; a fibrous material such as nonwoven fabric and cloth; and a metal foil.
In addition, a laminate sheet of a plastic film and a paper or a laminate sheet of a plastic film and a metal foil may be also employed.
From the standpoint that a lot of kinds of industrial products are present and many products having a thickness appropriate for use in a patch are commercially available, which makes it easy to select a material having the required quality, a polyester film, particularly, a polyethylene terephthalate film, is preferred.

In view of easy processing applicability or processing precision, the release liner 30 preferably has a uniform thickness.
The thickness of the release liner 30 is not particularly limited, but in view of easy production of the patch 1, cost of the release liner 30, and portability, operability and the like of the patch 1, the thickness is preferably from 25 to 200 µm, more preferably from 50 to 150 µm.
In order to make the release liner 30 more easily separable from the pressure-sensitive adhesive layer 20, a material where the side coming into contact with the pressure-sensitive adhesive surface 20a has been subjected to a release treatment to form a lightly releasing surface is preferably employed as the release liner 30.

Also, the release liner 30 is preferably formed of a material exhibiting an elongation of less than 250% at the tensile test, and the percentage elongation of the patch 1 is also preferably less than 250%.
If the elongation of the release liner 30 is 250% or more, when a force is applied to the release liner for separating the release liner, the connection part 32 may be greatly deformed, making it difficult to split the release liner.

In the patch 1 of this embodiment, it is important for easily splitting the release liner 30 in use to form a parting line C in the release liner 30 by alternately providing cut lines 31 and connection parts 32, as described above.
Particularly, for facilitating the operation of sequentially cutting the connection parts 32 along the parting line C and separating (removing) the first region 30a from the pressure-sensitive adhesive surface 20a while splitting the release liner 30 to thereby remove the first region 30a in advance of removing the second region 30b, it is important to dispose the cut lines by aligning the downstream end parts along the splitting direction and arranging the upstream end part to be located on the first region side relative to the downstream end part so that at the time of dividing the first region from the second region, the connection part between one of the cut lines on the upstream side in the splitting direction and another one of the cut lines adjacent to the downstream side of the one of the cut lines can be cut in the form where the downstream end part of the one of the cut lines and a midway portion between the upstream end part and the downstream end part of the another one of the cut lines can be connected and the cutting direction connecting the downstream end part of the one of the cut lines and the midway portion of the another one of the cut lines can be inclined toward the first region (to the first region side) with respect to the splitting direction.

The above is described in detail by referring to Figs. 2A to 2C.
In this embodiment, a parting line C for guiding the cutting position when splitting the release liner 30 to create a desired split state is formed by linear cut lines 31.
Individual cut lines 31 are disposed to let their extending direction disagree with the splitting direction of the release liner 30 and be inclined with respect to the splitting direction and at the same time, to run substantially in parallel with each other.
The cut lines 31 are formed by putting their downstream end parts into a state of being aligned substantially on one straight line along the splitting direction and moreover, arranging the downstream end parts nearly at equal intervals.
Also, all cut lines are substantially the same in terms of the length and the tilt angle with respect to the splitting direction.
Accordingly, the upstream end parts of the cut lines 31 are aligned substantially on one straight line on the first region side relative to the downstream end parts and arranged nearly at equal intervals.

The term "substantially on one straight line" as used herein does not strictly mean only a case where a line connecting the downstream end parts or upstream end parts of all cut lines forms one straight line, but is intended to include also a case where those end parts are located in a linear (belt-like) region having a certain degree of width.
However, if an excessive width is set for the belt-like region, this state is not "on one straight line" any more. Therefore, the width of the belt-like region is usually 10 mm or less.
That is, as long as it is in the linear region having a width of 10 mm, even when the cut lines are disposed in a gently curving manner or slightly in a zigzag manner within the region, this can be regarded as "substantially on one straight line".

In the patch 1 according to this embodiment, the parting line C is preferably provided so that the first region 30a and the second region 30b can be divided from each other to have substantially equal areas, because the user usually splits the release liner 30 by separately holding the first region 30a and the second region 30b of the release liner 30 by left and right hands.
The term "substantially equal areas" as used herein means that the ratio of the area of one region to the area of the other region is from 80 to 120%.

In the cut line 31, the maximum width in the direction orthogonal to the incision direction is preferably from 1 to 120 µm.
If the maximum width of the cut line is excessively large, a component forming the pressure-sensitive adhesive layer 20 may leak out from the cut line.
On the other hand, it may be difficult from a production-technical viewpoint to form, in the release liner, a cut line whose maximum width is excessively small.

The total length of one cut line may be, for example, any in the range of 0.1 to 60 mm, preferably any in the range of 1.0 to 20 mm.
If the total length of the cut line is excessively large, a component forming the pressure-sensitive adhesive layer 20 may leak out from the cut line.
On the other hand, if the total length of the cut line is too small, the connection part between cut lines tends to become relatively large, requiring a force to form a rupture line in the connection part and thereby form a parting line, and a great force may be required for separating the release liner.

The cut line 31, the connection part 32 and the cutting method for the connection part 32 are described in more detail by referring to Fig. 2B.
First, in Fig. 2B, when notice is taken of three cut lines aligning from the upstream side to the downstream side in the splitting direction (referred to in sequence as first cut line 31 a, second cut line 31 b, and third cut line 31c), as described above, the first cut line 31 a, the second cut line 31 b and the third cut line 31c are disposed substantially in parallel with each other.
Moreover, these cut lines are constituting the parting line C by inclining their length direction with respect to the splitting direction D1.
Accordingly, the angle made by a line segment connecting the downstream end part 31a" of the first cut line 31a and the downstream end part 31b" of the second cut line 31b, with the first cut line 31a, and the angle made by the line segment above with the second cut line 31b, are nearly the same.
The term "substantially in parallel" as referred to herein is used with intent to include, in addition to a strictly parallel state, a case where lines are slightly deviated from the strictly parallel state, for example, the distance between lines fluctuates by about ±10%.
Accordingly, there may be a case where the angle between the first cut line 31 a and the line segment above and the angle between the second cut line 31b and the line segment above differ in the above-described fluctuation range.
The same applies to the relationship between the second cut line 31b and the third cut line 31c.
The acute angle of the angles made by each cut line with the line segment above is usually from 5 to 80°, preferably from 10 to 70°.

If such an angle is excessively large, the difference between the direction of a force applied to the release liner by the user and the cut line direction is increased, and this may make it difficult to smoothly separate the release liner.
On the other hand, if the above-mentioned angle is too small, the connection part between cut lines tends to become relatively small, giving high probability that the connection part is deformed, and this may lead to a fear of enlarging the width of the cut line upon application of a stress and leaking a component of the pressure-sensitive adhesive layer from the cut line.

The distance from the downstream end part of one of the cut lines aligning substantially on one straight line to the nearest position of the adjacent cut line is, for example, from 0.01 to 100 mm, preferably from 0.1 to 10 mm, particularly preferably from 0.5 to 5.0 mm.
If this distance is excessively large, the connection part between cut lines tends to become relatively large, requiring a strong force to cut the connection part, and a great force may be required for separating the first region 30a of the release liner 30.
On the other hand, if the above-mentioned distance is too small, the connection part 32 between cut lines 31 tends to become relatively small, giving high probability that the connection part 32 is deformed upon application of a stress to the patch 1, and this is likely to let the width of the cut line 31 be enlarged and a component forming the pressure-sensitive adhesive layer 20 be leaked from the cut line 31.

In the patch 1 according to this embodiment, each connection part 32 is present between two of the cut lines 31 aligning substantially on one straight line as described above and the connection parts 32 are cut by the user sequentially from the upstream side to the downstream side in the splitting direction.
As a result, the release liner 30 is halved and split into a first region 30a and a second region 30b.
Incidentally, Figs. 2A to 2C shows a patch 1 where the operation of sequentially cutting the connection parts 32 along the parting line while separating a part of the release liner 30 is performed easily for a right-handed user.
Accordingly, the region on the right of the front view of Fig. 2A is the first region 30a separated first, and the region on the left is the second region 30b.

At this time, the user usually performs the operation of separating the first region 30a by the right hand while grasping the second region 30b by the left hand.
Therefore, it is an easier operation for the user to apply the force to the connection part 32 not in the vertical direction of the front view of Fig. 2B but in the slightly obliquely downward right (arrow D2) direction, that is, in the direction inclined toward the first region.
In this embodiment, from the first cut line 31 a to the third cut line 31c are disposed so that the downstream end parts are aligned along the splitting direction and each upstream end part is located on the first region side relative to the corresponding downstream end part. Therefore, at the time of cutting, for example, the connection part 32 between the first cut line 31a and the second cut line 31b adjacent to the downstream side of the first cut line 31a, the connection part 32 can be cut in a form where a starting point is at the downstream end part 31 a" of the first cut line 31 a and a terminal point is at a midway portion between the upstream end part 31b' and the downstream end part 31b" of the second cut line 31b and moreover, the cutting direction of the connection part 32 can be put into a state of being inclined toward the first region 30a with respect to the splitting direction D1.
That is, the connection part 32 can be cut in a cutting state denoted by a numeral 33 in Fig. 2B and thus, the connection part 32 can be cut in the direction where cutting is easy for the user.

Also, at the time of subsequently cutting the connection part between the second cut line 31b and the third cut line 31c, since the second cut line 31b is disposed by arranging its downstream end part 31b" along the splitting direction, similarly to the first cut line 31a, and the upstream end part 31 c' of the third cut line 31c is disposed on the first region side, the connection part 32 can be cut in a form where a starting point is at the downstream end part 31b" of the second cut line 31b and a terminal point is at a midway portion between the upstream end part 31c' and the downstream end part 31c" of the third cut line 31c and thus, the connection part 32 can be cut in the direction close to the direction (D2) where a force is easily put on by the dominant hand.

In this way, in the operation of separating the first region 30a, all connection parts can be cut in the direction where a force is easily put on by the dominant hand.
The same applies to a release liner where three or more cut lines are aligned, and in a case where the release liner has N pieces of cut lines, the same operation as the operation described regarding the cutting method of the connection parts from the first cut line 31a to the third cut line 31c is repeated for the (n-1)-th cut line, the (n)-th cut line and the (n+1)-th cut line (wherein N is a natural number of 3 or more, and n is a natural number of 2 or more), whereby the first region 30a can be divided from the second region 30b and separated from the pressure-sensitive adhesive surface 20a.

Incidentally, when the connection part is cut by a right-handed user and the force is applied in a natural manner, the cutting direction ("θ" in Fig. 2B) of the connection part is usually inclined with respect to the splitting direction D1 at approximately from 10 to 50° toward the first region.
Accordingly, for forming a release liner 30 which can be easily split in particular by a right-handed user, it is preferred to adjust, for example, the shape of individual cut lines or the distance between adjacent cut lines and thereby form a parting line so that the (n+1)-th cut line can be present between a line segment extending from the downstream end part of the (n)-th cut line and in the direction inclined at 10° toward the first region with respect to the splitting direction D1 and a line segment extending from the downstream end part of the (n)-th cut line and in the direction inclined at 50° toward the first region with respect to the splitting direction D1.
From the standpoint of more reliably obtaining such an effect, the upstream end part of one of the cut lines adjacent to the downstream side of another one of the cut lines is preferably located on the upstream side in the splitting direction relative to the downstream end part of the another one of the cut lines.

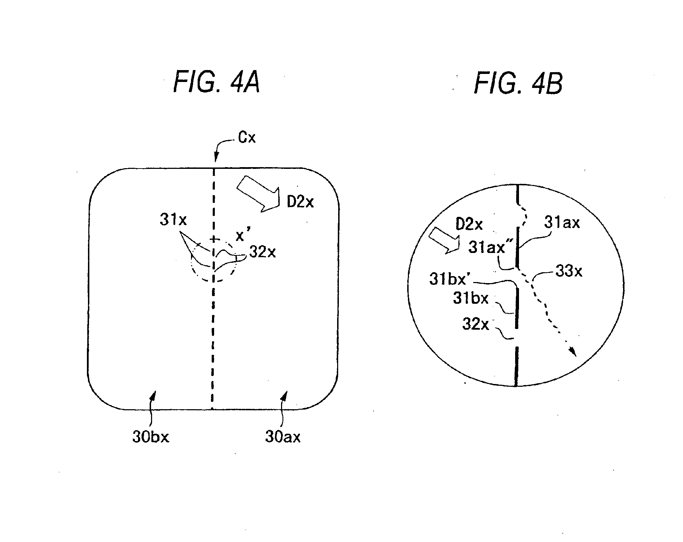

The effect above is described in more detail by referring to Figs. 4A and 4B showing a patch 1x which is outside the scope of the present invention. In a case where a linear dashed parting line Cx called "perforation" is formed by cut lines 31x and connection parts 32x, when the connection part is cut from the downstream end part 31ax" of the first cut line 31 ax toward the direction D2x where a force is easily applied by the user, this cutting is liable to result in the state denoted by numeral 33x in the figures and since the second cut line 31 bx is not present in this cutting direction, it becomes difficult to split the release liner 30x along the parting line Cx.
Accordingly, in the patch 1x shown in Figs. 4A and 4B, the manner of applying a force when splitting the release liner 30x by the user must be devised, and a careful work is required.

On the other hand, in the patch 1 of this embodiment, as described above, the splitting operation can be easily practiced.
Furthermore, in the patch 1 of this embodiment, a parting line C is formed in the release liner 30 by alternately providing cut lines 31 and connection parts 32. Therefore, unlike the patch described in International Publication No. WO00/69422 mentioned above, the cut line can be kept from being largely opened due to slight stress to greatly expose the pressure-sensitive adhesive surface through the cut line.
In addition, according to the patch described in JP-A-2008-19209 mentioned above, unless the depth of the groove is strictly controlled, the release liner can be hardly split in a desired state. On the other hand, the patch 1 according to this invention enjoys easy production in that the release liner capable of being split in a desired state can be formed only by providing predetermined cut lines.
Incidentally, these effects are not limited to the patch 1 illustrated above as an embodiment.

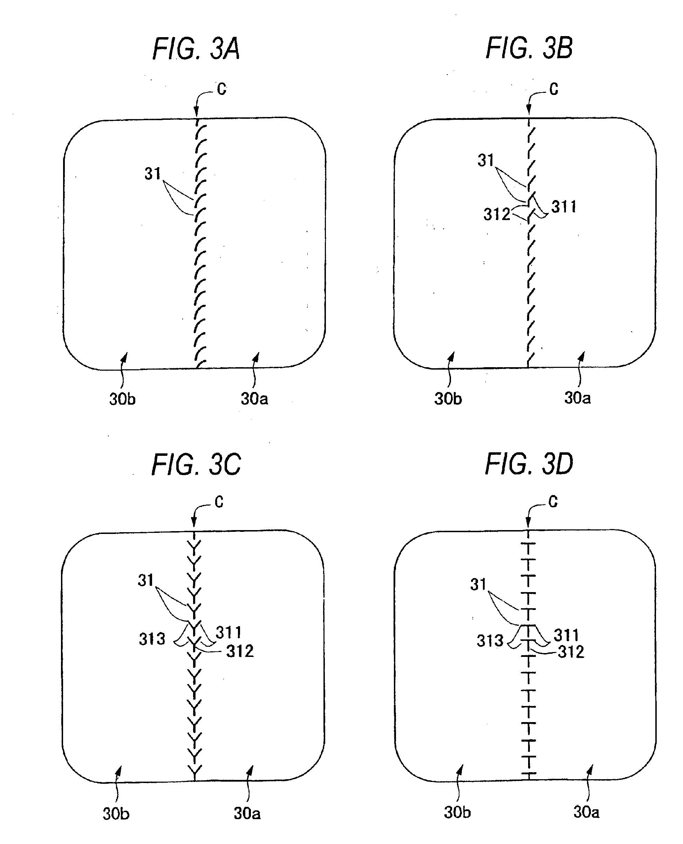

Other embodiments of the patch of the present invention are described below by referring to Figs. 3A to 3D showing other embodiments of the manner of providing the parting line C (cut lines 31).
The patch 1 shown in Fig. 3A is the same as the patch 1 illustrated in Figs. 2A to 2C in that the downstream end parts are arranged along the splitting direction and at the same time, each upstream end part is located on the first region side relative to the corresponding downstream end part, and also the same as the patch illustrated in Figs. 2A to 2C in that at the time of dividing the first region 30a from the second region 30b, the connection part between one of the cut lines on the upstream side in the splitting direction and another one of the cut lines adjacent to the downstream side of the one of the cut lines above can be cut in a form where the downstream end part of the one of the cut lines and a midway portion between the upstream end part and the downstream end part of the another one of the cut lines can be connected and the cutting direction connecting the downstream end part of the one of the cut lines and the midway portion of the another one of the cut lines can be inclined toward the first region with respect to the splitting direction.
On the other hand, while a linear cut line extending from the upstream end part toward the downstream end part is formed in the patch illustrated in Figs. 2A to 2C, a rounded cut line is formed in the patch 1 shown in Fig. 3A.
More specifically, in the patch illustrated in Figs. 2A to 2C, a linear cut line inclined toward the second region is formed when viewed from the upstream end part toward the downstream end part, whereas in the patch 1 shown in Fig. 3A, an arced cut line is formed by causing the portion between the starting point (upstream end part) and the terminal point (downstream end part) of the cut line to bulge toward the second region 30b.

The patch 1 shown in Fig. 3A also has the same effects as the patch shown in Figs. 2A to 2C. Moreover, in the patch shown in Figs. 2A to 2C, the angle between the cut line and the parting line C is not changed until the downstream end part of the cut line, whereas in the patch 1 shown in Fig. 3A, a part of the cut line close to the downstream end part is in a state of running along the direction in which the parting line C extends (the angle with respect to the splitting direction is close to 0°) and therefore, the operation of splitting the release liner 30 can be more smoothly performed.
These effects are the same also in the patch 1 illustrated in Fig. 3B described below.

In the patch 1 shown in Fig. 3B, the cut line 31 is composed of two segments (portions) and of these, one segment 311 has the same configuration as the cut line 31 of the patch 1 shown in Figs. 2A to 2C.
That is, in the patch shown in Figs. 2A to 2C, the cut line is composed of only a linear portion running in the oblique direction with the starting point locating at the upstream end part and the terminal point locating at the downstream end part, whereas in the patch 1 shown in Fig. 3B, the cut line 31 is composed of a portion running in the oblique direction from the upstream end part toward the downstream side and a remaining portion (hereinafter, the portion running in the oblique direction from the upstream end part toward the downstream side is sometimes referred to as a "first segment").

More specifically, in the patch 1 shown in Fig. 3B, the terminal point of the first segment 311 is located at a point on the upstream side traced back from the downstream end part of the cut line 31 in the direction opposite the splitting direction, and the cut line further has, other than the first segment, a linear second segment extending from the terminal point of the first segment to the downstream end part.
In the patch 1 shown in Fig. 3B, similarly to the patch 1 illustrated in Figs. 2A to 2C, the first segments 311 are preferably disposed substantially in parallel with each other.
The acute angle of the angles made by the first segment 311 with respect to the splitting direction is, for example, from 5 to 90°, preferably from 10 to 90°.
According to the patch 1 shown in Fig. 3B, compared with the embodiment of Figs. 2A to 2C which does not have a second segment, the same effects as in the embodiment of Figs. 2A to 2C can be expected even when the angle of the first segment 311 is increased.
This is because owing to the possession of a second segment 312 along the parting line direction, the operation of separating the first region 30a while performing the splitting by the parting line C becomes smooth.

In a case where, as shown in Fig. 3B, the cut line 31 has a further segment (second segment) in addition to the first segment 311, each of the first segment 311 and the second segment 312 has a length of, for example, from 0.5 to 30 mm, preferably from 0.5 to 10 mm.
In the patch 1 shown in Fig. 3B, compared with the embodiment illustrated in Figs. 2A to 2C which does not have a second segment in the cut line, the same effects as in the embodiment illustrated in Figs. 2A to 2C can be expected even when the length of the first segment 311 is shortened.
This is considered because thanks to the presence of the further segment, a part of the roles of the first segment 311 can be taken by that segment.

It is the same as above with respect to the point that if the total length of one cut line is excessively large, a component of the pressure-sensitive adhesive layer may leak and the point that if the total length thereof is too small, the connection part tends to become relative large and a great force is required for cutting the connection part.
Accordingly, also in the patch 1 shown in Fig. 3B, the total length of one cut line is usually from 0.1 to 60 mm, preferably from 1.0 to 20 mm, and this is the same as in the patch described hereinbefore.
In Fig. 3B, the angle between the first segment and the second segment is, for example, from 90 to 175°, preferably from 90 to 170°.
The distance from the downstream end part of one of the cut lines disposed substantially on one straight line to the nearest position of the adjacent one of the cut lines is, for example, from 0.01 to 100 mm, preferably from 0.1 to 10 mm, particularly preferably from 0.5 to 5.0 mm.

If such an angle or a distance is excessively large, the connection part between cut lines tends to become relatively large, requiring a strong force to cut the connection part, and a great force may be required for separating the first region 30a.
On the other hand, if the angle or distance above is too small, the connection part 32 between cut lines 31 tends to become relatively small, giving high probability that the connection part 32 is deformed, and this is likely to let the width of the cut line 31 be enlarged and a component forming the pressure-sensitive adhesive layer 20 be leaked from the cut line 31.

The patch 1 shown in Fig. 3C is described below.
The cut line 31 of the patch 1 shown in Fig. 3C is the same as the cut line of the patch shown in Fig. 3B in that the cut line has a first segment 311 locating the starting point at the upstream end part and the terminal point at a point traced back from the downstream end part in the direction opposite the splitting direction and further has a second segment 312 extending from the terminal point of the first segment 311 to the downstream end part.
On the other hand, the patch 1 shown in Fig. 3C differs from the patches 1 illustrated hereinbefore in that a third segment 313 locating the starting point at the terminal point of the first segment 311 and the terminal point on the upstream side relative to the terminal point of the first segment 311 and at the same time, on the second region side opposite the first segment is further provided and the first segment 311, the second segment 312 and the third segment 313 are disposed in a Y-shaped manner.

Incidentally, the patch illustrated in Figs. 2A to 2C is excellent in that even when the cutting of the connection parts is started from whichever side out of both ends of the parting line C, the same effect can be obtained, because the shape of the cut line has a rotational symmetry (twofold symmetry). On the other hand, the patch 1 shown in Fig. 3C has a line-symmetric shape with respect to the parting line C and therefore, is excellent in that even when the user is not right-handed but left-handed, the same effect can be obtained.
That is, in Figs. 2A to 2C and Figs. 3A and 3B, since a user whose dominant hand is the right hand is envisaged, the right side of the front view is taken as the region separated first (first region 30a) of the release liner 30.
On the other hand, in the case of envisaging that a left-handed user first separates the first region by his dominant hand, the observer's left side of the release liner becomes the first region and the cut line shown in Figs. 2A to 2C and Figs. 3A and 3B needs to be formed by reversing the left-right arrangement, but in Fig. 3C, the cut line is originally line-symmetric and therefore, such switching is not necessary.
That is, in the patch 1 shown in Fig. 3C, the third segment 313 of the cut line can function in the same manner as the first segment 311 in Figs. 2A to 2C and Figs. 3A and 3B, and the patch 1 can be a patch for both a right-handed user and a left-handed user.

In the embodiment shown in Fig. 3C, the length of the third segment is, for example, from 0.5 to 30 mm, preferably from 0.5 to 10 mm.

In the embodiment shown in Fig. 3C, the angle between the first segment 311 and the second segment 312 is, for example, from 90 to 175°, preferably from 90 to 170°.
Also, the angle between the third segment 313 and the second segment 312 is, for example, from 90 to 175°, preferably from 90 to 170°.
The angle between the first segment 311 and the third segment 313 is preferably from 10 to 180°, more preferably from 20 to 180°.

These angles are preferred for the same reasons as in the patch illustrated in Fig. 3B.
Furthermore, preferred embodiments of the width and total length of the cut line can be selected based on the reasons described hereinbefore.
The distance from the downstream end part of one cut line to the nearest position of the adjacent cut line is, for example, from 0.01 to 100 mm, preferably from 0. to 10 mm, more preferably from 0.5 to 5.0 mm.

Incidentally, in addition to the Y-shaped cut line of Fig. 3C, usability for both a left-handed user and a right-handed user is attained also in the case of providing a T-shaped cut line shown in Fig. 3D, and the same effect as in the patch illustrated in Fig. 3C can be expected also in the embodiment shown in Fig. 3D.

The production method of the patch described above is not particularly limited but can be performed, for example, by a following method.
A release liner is first prepared, a pressure-sensitive adhesive layer is stacked on one surface of the release liner, and a backing is stacked on the pressure-sensitive adhesive layer.
Alternatively, a backing is prepared, a pressure-sensitive adhesive layer is stacked on one surface of the backing, and a release liner is stacked on the pressure-sensitive adhesive layer.
The method for stacking the pressure-sensitive adhesive layer is not particularly limited and includes a method of coating and adhering a composition for formation of the pressure-sensitive adhesive layer.
Also, only a pressure-sensitive adhesive layer may be separately produced and welded to a backing or a release liner.
The method for imparting a cut line having the above-described shape to the release liner includes known techniques, for example, a processing with a blade such as die roll and razor, and a laser processing.

### Examples

The present invention is described in greater detail below by referring to specific examples

### (Example 1)

### <Preparation of Composition for Forming Pressure-Sensitive Adhesive Layer>

Polyisobutylene 1 having a high molecular weight (viscosity average molecular weight: 4,000,000), polyisobutylene 2 having a low molecular weight (viscosity average molecular weight: 55,000), a tackifier (alicyclic saturated hydrocarbon resin, softening point: 100°C (ring-and-ball method)), an organic liquid component (isopropyl myristate) and isosorbide dinitrate as a drug were mixed in a mass ratio of 15/25/25/30/5 in the presence of toluene to obtain a composition for forming the pressure-sensitive adhesive layer.

### <Production of Pressure-Sensitive Adhesive Sheet>

On the lightly releasing surface of PET film (thickness: 75 µm) release-treated to let one surface work as a lightly releasing surface, the composition prepared above was coated to form a pressure-sensitive adhesive layer having a dry thickness of 200 µm and dried by a dryer (100°C, 5 minutes), whereby a PET film having formed thereon a pressure-sensitive adhesive layer was obtained.

Furthermore, a laminate sheet (total thickness: about 40 µm) working out to a backing was stacked on the pressure-sensitive adhesive layer to produce a pressure-sensitive adhesive sheet used as the raw material of patches of Examples and Comparative Examples.

Incidentally, as the laminate sheet working out to a backing, a sheet where a PET-made film having a thickness of 3.5 µm and a PET-made nonwoven fabric having a thickness of about 35 µm and a basis weight of 12 g/m² are stacked, was used, and the PET nonwoven side was abutted on the pressure-sensitive adhesive layer to form the pressure-sensitive adhesive sheet.

### <Production of Cut Line on Release Liner>

As the member for forming the release liner, a PET film (thickness: 75 µm) having formed thereon a lightly releasing surface similarly to the PET film used for formation of the pressure-sensitive adhesive sheet was prepared, and a parting line in the embodiment shown in Figs. 2A to 2C was formed thereon.
That is, a parting line was formed on the PET film by 5 mm-long linear cut lines each consisting of only a first segment.
Each cut line was formed to make an angle of 20° with the splitting direction.
Also, the cut lines were arranged such that the shortest distance from the downstream end part of one of the cut lines to another one of the cut lines adjacent to the downstream side of the one of the cut lines became 1.5 mm.

### <Punching Out of Patch>

The PET film already laminated to the pressure-sensitive adhesive sheet was separated and instead, the PET film for formation of a release liner, where cut lines were formed as above, was stacked on the pressure-sensitive adhesive layer, and the resulting sheet was used as the raw material sheet of the patch.

Incidentally, this raw material sheet was formed in a form of laminating the lightly releasing surface side of the PET film to the pressure-sensitive adhesive surface.

Subsequently, the raw material sheet was punched out in a square of 60 mm x 60 mm by using a Thompson blade to obtain the patch of Example 1.

Here, punching out by a Thompson blade was performed such that the cut lines were aligned along a line segment connecting midpoints of opposing two sides to each other in the square.

That is, the patch was produced so that when the release liner was split into two halves by cutting the connection parts between cut lines, the release liner could be divided into two equal parts.

Moreover, the raw material sheet was punched out to cut the midway portion of the cut line so that the incision becoming a lead for the operation of splitting the release liner could be formed at the outer edge part of the release liner.

### (Example 2)

The patch of Example 2 was obtained in the same manner as in Example 1 except that the shape of the cut line formed was changed from the embodiment shown in Figs. 2A to 2C to the embodiment shown in Fig. 3B.
That is, a square patch of 60 mm x 60 mm was produced using, as the release liner, a PET film where a parting line was formed by cut lines each having a first segment locating the starting point at the upstream end part and the terminal point at a point traced back from the downstream end part in the direction opposite the splitting direction and a second segment extending from the terminal point of the first segment to the downstream end part.
Incidentally, the length of the first segment was set to 2.1 mm, and the angle (acute angle) of the first segment with respect to the splitting direction by the parting line was set to 45°.
In addition, the second segment was formed to linearly extend from the terminal point of the first segment to the downstream end part of the cut line along the splitting direction.
That is, the cut line was formed to make an angle of 135° between the first segment and the second segment.
Here, the length of the second segment was set to 3.5 mm.
Furthermore, in the patch of Example 2, cut lines were provided on the release liner such that the shortest distance from the downstream end part of one of the cut lines to another one of the cut lines adjacent to the downstream side of the one of the cut lines became 1.1 mm.

### (Example 3)

The patch of Example 3 was obtained in the same manner as in Example 1 except that the shape of the cut line formed was changed from the embodiment shown in Figs. 2A to 2C to the embodiment shown in Fig. 3C.
That is, a square patch of 60 mm x 60 mm was produced using, as the release liner, a PET film where a parting line was formed by cut lines each having a first segment locating the starting point at the upstream end part and the terminal point at a point traced back from the downstream end part in the direction opposite the splitting direction and a second segment extending from the terminal point of the first segment to the downstream end part and further having a third segment locating the starting point at the terminal point of the first segment and the terminal point on the upstream side relative to the starting point and at the same time, on the second region side opposite the first segment, in which the first segment, the second segment and the third segment were disposed in a Y-shaped manner.
Incidentally, the length and angle of the first segment, the length of the second segment, and the like were the same as those in Example 2.
That is, the patch of Example 3 was the same as the patch of Example 2 except that a third segment was provided in the cut line.
Here, the third segment was in a line-symmetric relationship with the first segment, and the length thereof was the same as that of the first segment.
That is, the third segment was formed to have a length of 2.1 mm and make an angle of 135° with the second segment and an angle of 90° with the first segment.

### (Comparative Example 1)

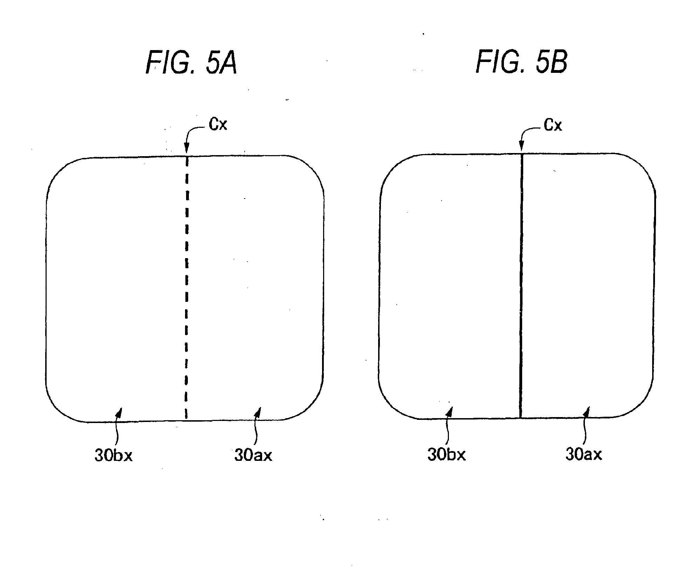

The patch of Comparative Example 1 was produced in the same manner as in Example 1 except that a dashed parting line shown in Fig. 5A was formed on the release liner.
That is, the parting line was formed by alternately providing a 2.0 mm-long cut lines linearly formed along the splitting direction and connection parts each having a length of 1.0 mm.

### (Comparative Example 2)

As shown in Fig. 5B, along a line segment connecting midpoints of opposing two sides to each other in a square patch, a cut line continuing from one end to the other end of the line segment was formed on a release liner.
That is, in the patch of Comparative Example 2, the pressure-sensitive adhesive surface was covered with a release liner which was previously split into two halves.

### (Evaluation)

The patches of Examples 1 to 3 and Comparative Examples 1 and 2 were evaluated for the following evaluation items.

### (Test Example 1)

### <Measurement of Width of Cut Line of Release Liner>

The cut line portion(s) of the release liner of the patch was observed by a digital microscope (VHX-600, manufactured by Keyence Corporation, magnification: 1,000 times), and out of all cut lines on the release liner of each patch, the portion where the width of the cut line was widest was measured.
The results are shown in Table 1.

### (Test Example 2)

### <Evaluation of Bleeding of Pressure-Sensitive Adhesive Layer Component After Storage and Removability from Packaging>

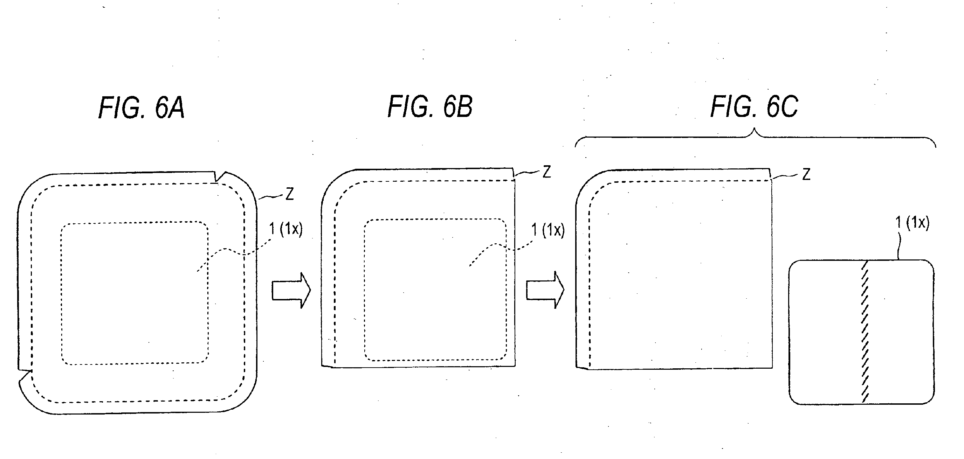

As shown in Fig. 6A, each of the patches 1 (1x) of Examples 1 to 3 and Comparative Examples 1 and 2 was sealed in a packaging bag Z formed of a packaging material (outer dimension: 100 mm x 100 mm, inner dimension: 90 mm x 90 mm) where the outer layer was composed of a 12 µm-thick PET film and the inner layer was composed of a 30 µm-thick polyacrylonitrile-based resin film.
The packaging bag Z having sealed therein the patch 1 (1x) was horizontally placed on a stand and in the state of a load of about 300 gf being applied, stored in a constant-temperature vessel at a temperature of 50°C for 2 weeks.
After the storage, as shown in Fig. 6B, two sides of the packaging bag Z were cut by scissors to open the bag.
From the opened portion, fingers were inserted and the patch 1 (1x) was taken out by gripping the corner. The bleeding of a component forming the pressure-sensitive adhesive layer from the cut line of the release liner and the removability of the patch from the packaging were evaluated using scores 1 to 5 according to the following evaluation standards.
The results are shown in Table 1.

### <Evaluation Standards>

5: There is no bleeding of a component of the pressure-sensitive adhesive layer, and the patch can be very easily taken out.
4: There is slight bleeding of a component of the pressure-sensitive adhesive layer, but the patch can be very easily taken out.
3: There is some bleeding of a component of the pressure-sensitive adhesive layer, but the patch can be easily taken out.
2: There is some bleeding of a component of the pressure-sensitive adhesive layer, but the patch can be taken out.
1: There is significant bleeding of a component of the pressure-sensitive adhesive layer, and the patch can be hardly taken out.

### (Test Example 3)

### <Evaluation of Separability of Release Liner>

While dividing a part of the release liner along the parting line from the patch by four panelists, the divided region was separated from the pressure-sensitive adhesive surface to perform sensory evaluation of separability in the separating operation. Evaluation results are summarized in accordance with the following criteria. The results are shown in Table 1.

### <Summary Criteria of Evaluation>

- 5:: All of four persons felt easily separable.
- 4:: Three out of four persons felt easily separable.
- 3:: Two out of four persons felt easily separable.
- 2:: One out of four persons felt easily separable.
- 1:: All of four persons felt not easily separable.

**Table 1**

| | Total Length of Cut Line of Release Liner (mm) | Evaluation Results | | |
|---|---|---|---|---|
| | | Maximum Width of Cut Line of Release Liner (µm) | Bleeding of Component of Pressure-Sensitive Adhesive Layer After Storage and Removability from Packaging | Separability of Release Liner |
| Example 1 | 5.0 | 72 | 3 | 4 |
| Example 2 | 5.6 | 30 | 4 | 5 |
| Example 3 | 7.7 | 33 | 4 | 5 |
| Comparative Example 1 | 2.0 | 24 | 5 | 2 |
| Comparative Example 2 | 60.0 | 40 | 1 | 5 |

It is seen that according to the present invention, a patch ensuring that the pressure-sensitive adhesive surface can be kept from being largely exposed due to slight stress before use and the release liner can be easily split when using the patch, can be provided.

## Claims

1. A sheet-shaped patch (1) comprising: a backing (10); a pressure-sensitive adhesive layer (20) provided on one surface of the backing (10), the pressure-sensitive adhesive layer (20) providing a pressure-sensitive adhesive surface (20a) showing an adhesiveness at ordinary temperature; and a release liner (30) covering the pressure-sensitive adhesive surface (20a), having a parting line (C) constituted of cut lines (31) and connection parts (32) provided alternately, and being formed to be splittable into a first region (30a) and a second region (30b) by sequentially cutting the connection parts (32) along the parting line (C), thereby enabling separation of the first region (30a) from the pressure-sensitive adhesive surface ahead of the second region (30b) while performing cutting of the connection parts (32),
wherein the cut lines (31) each have an upstream end part and a downstream end part in a splitting direction (D1), and the cut lines (31) are disposed such that said downstream end parts are aligned along the splitting direction (D1) and each upstream end part is arranged to be located on a first region side relative to each corresponding downstream end part, so that, at the time of splitting the first region (30a) from the second region (30b), one of the connection parts (32) which is located between one of the cut lines (31) located on an upstream side in the splitting direction (D1) and another one of the cut lines adjacent to a downstream side of said one of the cut lines (31) can be cut in a form where the downstream end part of said one of the cut lines (31) and a midway portion between the upstream end part and the downstream end part of the another one of the cut lines (31) are connected and a cutting direction connecting the downstream end part of said one of the cut lines (31) and the midway portion of said another one of the cut lines (31) can be inclined toward the first region (30a) with respect to the splitting direction (D1).

2. The patch as claimed in claim 1, wherein each of the cut lines (31) includes a first segment (311) locating a starting point at the upstream end part and a terminal point at the downstream end part or at a point traced back from the downstream end part in a direction opposite the splitting direction, and the cut lines (31) are disposed such that said first segments (311) of the cut lines (31) adjacent to each other are provided substantially in parallel.

3. The patch as claimed in claim 2, wherein each of the cut lines (31) includes the first segment (311) locating the starting point at the upstream end part and a terminal point at the point traced back from the downstream end part in the direction opposite the splitting direction (D1) and further includes a second segment (312) extending from the terminal point of the first segment (311) to the downstream end part.

4. The patch as claimed in claim 3, wherein each of the cut lines (31) further includes a third segment (313) locating a starting point at the terminal point of the first segment (311) and a terminal point on an upstream side relative to the starting point thereof and also on the second region (30b) side opposite the first segment (311); and the first segment (311), the second segment (312) and the third segment (313) are disposed in a Y-shaped manner.

5. The patch as claimed in any one of claims 1 to 4, wherein each of the cut lines (31) has a maximum width of from 1 to 120 µm.

6. The patch as claimed in any one of claims 1 to 5, wherein each of the cut lines (31) has a total length of from 0.1 to 60 mm.

7. The patch as claimed in any one of claims 1 to 6, wherein the parting line (C) enables splitting the release liner (30) into substantially equal areas.

## Patentansprüche

1. Blattförmiges Pflaster (1) umfassend: eine Verstärkung bzw. einen Träger (10), eine druckempfindliche Haftschicht (20), welche auf einer Oberfläche der Verstärkung (10) bereitgestellt ist, wobei die druckempfindliche Haftschicht (20) eine druckempfindliche Haftfläche (20a) bereitstellt, die ein Klebevermögen bei normaler Temperatur zeigt; und eine Trennfolie (30), welche die druckempfindliche Haftfläche (20a) bedeckt, mit einer Trennungslinie (C), welche aus Schnittlinien (31) und Verbindungsteilen (32) besteht, die abwechselnd bereitgestellt sind, und so ausgebildet ist, dass sie in einen ersten Bereich (30a) und einen zweiten Bereich (30b) teilbar ist, indem die Verbindungsteile (32) entlang der Trennungslinie (C) aufeinanderfolgend geschnitten werden, wodurch die Trennung des ersten Bereichs (30a) von der druckempfindlichen Haftfläche vor dem zweiten Bereich (30b) ermöglicht wird, während das Schneiden der Verbindungsteile (32) durchgeführt wird,
wobei die Schnittlinien (31) einen oberen Endteil und einen unteren Endteil in einer Spaltrichtung (D1) aufweisen und die Schnittlinien (31) so angeordnet sind, dass die unteren Endteile entlang der Spaltrichtung (D1) ausgerichtet sind und jeder obere Endteil angeordnet ist, um in einer ersten Bereichsseite relativ zu jedem entsprechenden unteren Endteil angeordnet zu sein, so dass, zum Zeitpunkt des Spaltens des ersten Bereichs (30a) von dem zweiten Bereich (30b) eines der Verbindungsteile (32), welcher zwischen einer der an einer oberen Seite in der Spaltrichtung (D1) angeordneten Schnittlinien (31) und einer anderen der Schnittlinien in der Nähe einer unteren Seite der einen der Schnittlinien (31) angeordnet ist, in einer Form geschnitten werden kann, in dem der untere Endteil der einen Schnittlinie (31) und ein mittlerer Bereich zwischen dem oberen Endteil und dem unteren Endteil einer anderen der Schnittlinien (31) verbunden sind, und eine Schnittrichtung, welche das untere Endteil der einen der Schnittlinien (31) und den mittleren Bereich der anderen der Schnittlinien (31) verbindet, in Richtung des ersten Bereichs (30a) in Bezug auf die Spaltrichtung (D) geneigt sein kann.

2. Pflaster nach Anspruch 1, wobei jede der Schnittlinien (31) ein erstes Segment (311) umfasst, welches einen Anfangspunkt an dem oberen Endteil und einem Anschlusspunkt an dem unteren Endteil oder an einem Punkt ausgehend von dem unteren Endteil in einer zu der Spaltrichtung entgegengesetzten Richtung festlegt und wobei die Schnittlinien (31) so angeordnet sind, dass die ersten Segmente (311) der Schnittlinien (31), welche einander benachbart sind, im Wesentlichen parallel bereitgestellt sind.

3. Pflaster nach Anspruch 2, wobei jede der Schnittlinien (31) ein erstes Segment (311) umfasst, welches den Anfangspunkt an dem oberen Endteil und einen Anschlusspunkt an dem Punkt ausgehend von dem unteren Endteil in einer zu der Spaltrichtung (D1) entgegengesetzt Richtung festlegt, und des Weiteren ein zweites Segment (312) umfasst, welches sich von dem Anschlusspunkt des ersten Segments (311) zu dem unteren Endteil erstreckt.

4. Pflaster nach Anspruch 3, wobei jede der Schnittlinien (31) des Weiteren ein drittes Segment (313) umfasst, welches einen Anfangspunkt an dem Anschlusspunkt des ersten Segments (311) und einen Anschlusspunkt an einer oberen Seite relativ zu dem Anfangspunkt und in der zweiten Bereichs(30b)-Seite gegenüberliegend dem ersten Segment (311) festlegt; und wobei das erste Segment (311), das zweite Segment (312) und das dritte Segment (313) in einer Y-förmigen Art angeordnet sind.

5. Pflaster nach einem der Ansprüche 1 bis 4, wobei jede der Schnittlinien (31) eine maximale Breite von 1 bis 120 µm aufweist.

6. Pflaster nach einem der Ansprüche 1 bis 5, wobei jede der Schnittlinien (31) eine Gesamtlänge von 0,1 bis 60 mm aufweist.

7. Pflaster nach einem der Ansprüche 1 bis 6, wobei die Trennlinie (C) das Spalten der Trennfolie (30) in im Wesentlichen gleiche Bereiche ermöglicht.

## Revendications

1. Timbre médical en forme de feuille (1) comprenant : un dorsal (10) ; une couche adhésive autocollante (20) appliquée sur une face du dorsal (10), la couche adhésive autocollante (20) procurant une surface adhésive autocollante (20a) présentant une adhésivité à température ambiante ; et une protection antiadhésive (30) couvrant la surface adhésive autocollante (20a), ayant une ligne de séparation (C) constituée de lignes de découpe (31) et de parties de connexion (32) réparties en alternance, et formée pour être divisée en une première région (30a) et une deuxième région (30b) par découpe séquentielle des parties de connexion (32) le long de la ligne de séparation (C), ce qui permet la séparation de la première région (30a) de la surface adhésive autocollante avant celle de la deuxième région (30b) tout en effectuant la découpe des parties de connexion (32),
où les lignes de découpe (31) ont chacune, une partie terminale amont et une partie terminale aval dans une direction de division (D1), et les lignes de découpe (31) sont disposées de sorte que lesdites parties terminales aval sont alignées sur la direction de division (D1) et chaque partie terminale amont est agencée pour être située dans un côté de la première région par rapport à chaque partie terminale aval correspondante, pour que au moment de la division de la première région (30a) depuis la deuxième région (30b), une des parties de connexion (32), qui se situe entre une des lignes de découpe (31) située sur un côté amont en la direction de division (D1) et une autre ligne de découpe adjacente à un côté aval de ladite une des lignes de découpe (31) peut être découpée de telle sorte que la partie terminale aval de ladite une des lignes de découpe (31) et la partie centrale entre la partie terminale amont et la partie terminale aval de l'autre ligne de découpe (31) sont connectées et une direction de découpe connectant la partie terminale aval de l'une des lignes de découpe (31) et la partie centrale de l'autre ligne de découpe (31) peut être inclinée vers la première région (30a) par rapport à la direction de division (D1).

2. Timbre médical selon la revendication 1, où chacune des lignes de découpe (31) comprend un premier segment (311) situant un point de départ sur la partie terminale amont et un point terminal sur la partie terminale aval ou sur un point remonté de la partie terminale aval en direction opposée à la direction de division, et les lignes de découpe (31) sont disposées de sorte que lesdits premiers segments (311) des lignes de découpe (31) adjacents les uns aux autres soient sensiblement parallèles.

3. Timbre médical selon la revendication 2, où chacune des lignes de découpe (31) comprend le premier segment (311) situant le point de départ sur la partie terminale amont et un point terminal sur le point remonté de la partie terminale aval en direction opposée à la direction de division (D1), et comprend en outre, un deuxième segment (312) s'étendant depuis le point terminal du premier segment (311) vers la partie terminale aval.

4. Timbre médical selon la revendication 3, où chacune des lignes de découpe (31) comprend en outre, un troisième segment (313) situant un point de départ sur le point terminal du premier segment (311) et un point terminal sur un côté amont par rapport au point de départ de celui-ci et également, sur le côté de la deuxième région (30b) opposé au premier segment (311) ; le premier segment (311), le deuxième segment (312) et le troisième segment (313) étant disposés en forme de Y.

5. Timbre médical selon l'une quelconque des revendications 1 à 4, où chacune des lignes de découpe (31) a une largeur maximale allant de 1 à 120 µm.

6. Timbre médical selon l'une quelconque des revendications 1 à 5, où chacune des lignes de découpe (31) a une longueur totale allant de 0,1 à 60 mm.

7. Timbre médical selon l'une quelconque des revendications 1 à 6, où la ligne de séparation (C) permet la division de la protection antiadhésive (30) en surfaces sensiblement égales.
